# EUROPEAN PATENT APPLICATION

(11) **EP 4 122 458 A1**
(43) Date of publication of application: **25.01.2023**
(21) Application number: 21771782.6
(22) Date of filing: 11.03.2021
(51) Int. Cl.: A61K 31/122, A61K 31/4406, A61P 25/00, A61P 25/16, A61P 25/28, A61P 43/00, C07C 69/67, C07D 213/80

(54) **MITOCHONDRIAL DYSFUNCTION IMPROVING AGENT**

(30) Priority: 17.03.2020 JP 2020046864
(71) Applicant: Takata, Jiro, Fukuoka 819-0044 (JP); Matsunaga, Kazuhisa, Fukuoka-shi, Fukuoka 814-0151 (JP); Karube, Yoshiharu, Fukuoka-shi, Fukuoka 814-0144 (JP); Iwasaki, Katsunori, Fukuoka-shi, Fukuoka 814-0121 (JP); Setoguchi, Shuichi, Fukuoka 814-0033 (JP); Terada, Kazuki, Hyogo 657-0835 (JP)
(72) Inventor: Takata, Jiro, Fukuoka 819-0044 (JP); Matsunaga, Kazuhisa, Fukuoka-shi, Fukuoka 814-0151 (JP); Karube, Yoshiharu, Fukuoka-shi, Fukuoka 814-0144 (JP); Iwasaki, Katsunori, Fukuoka-shi, Fukuoka 814-0121 (JP); Setoguchi, Shuichi, Fukuoka 814-0033 (JP); Terada, Kazuki, Hyogo 657-0835 (JP)
(74) Representative: Lippert Stachow Patentanwälte Rechtsanwälte
(86) International application number: PCT/JP2021/009811
(87) International publication number: WO 2021/187314

(57) **Abstract**

The object of the present invention is to provide a mitochondrial dysfunction improving agent comprising a vitamin K derivative having a high deliverability to mitochondria.

A mitochondrial dysfunction improving agent, neurodegeneration improving agent, amyotrophic lateral sclerosis improving agent, Alzheimer's disease improving agent, and Parkinson's disease improving agent comprising at least one of a carboxylic acid ester of an active vitamin K represented by a general formula (1) or a salt thereof, and 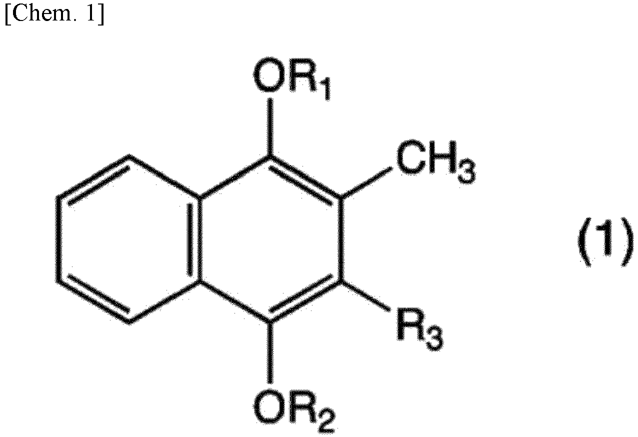 (wherein, R₁ and R₂ are a hydrogen atom, respectively, or a substituent selected from glycine, N-acyl glycine, N-alkyl glycine, N,N-dialkyl glycine, N,N,N-trialkyl glycine, acyl, dicarboxylic acid hemiester, and salts thereof; and at least either of R₁ and R₂ is glycine, N-acyl glycine, N-alkyl glycine, N,N-dialkyl glycine, N,N,N-trialkyl glycine, acyl, dicarboxylic acid hemiester, and salts thereof. R₃ is a group represented by a general formula (2), or a general formula (3). n is an integer of 1 to 7) 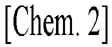 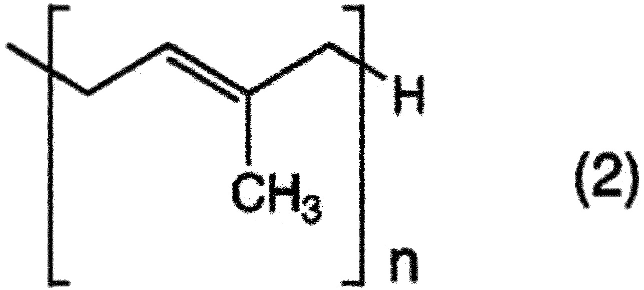 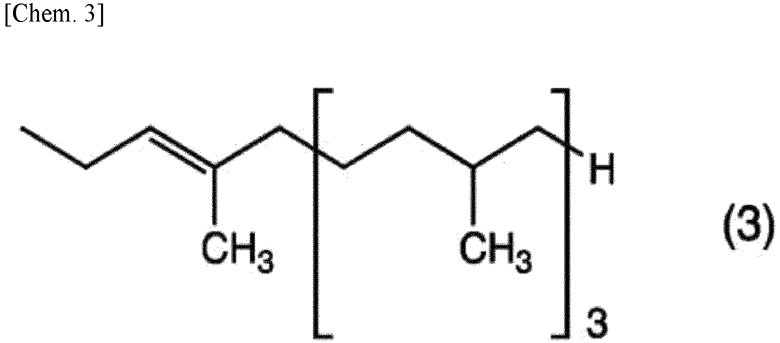 a mitochondrial dysfunction improving agent, neurodegeneration improving agent, amyotrophic lateral sclerosis improving agent, Alzheimer's disease improving agent, and Parkinson's disease improving agent comprising a carboxylic acid ester of an active vitamin K or a salt thereof (in the general formula (1), R₁ and R₂ is a carboxylic acid residue selected from a group consisting of R₄OOCCH₂CH₂CO- and R₄OOCCH₂CH₂CH₂CO-. R₃ represents the above general formula (2) or (3). R₄ is a C1-C3 alkyl group.).

## Description

### Related Application

The present application is based upon and claims the benefit of priority from Japanese Patent Application No. 2020-046864 filed on March 17, 2020, the entire contents of which are incorporated herein by reference.

### Technical Field

The present invention relates to a mitochondrial dysfunction improving agent, and particularly to a mitochondrial dysfunction improving agent having a vitamin K hydroquinone derivative as an active ingredient.

### Background Art

Mitochondrial dysfunction is considered to cause various diseases such as neurodegenerative diseases and type 2 diabetes.

For example, Alzheimer's disease (AD) is the most general neurodegenerative disease, and is a devastating disease pathologically characterized by strong aggregation of amyloid-β peptide (Aβ) and hyperphosphorylated tau (p-tau). There is a need for development of an effective and safe treatment method that targets at the AD lesion to suppress its progress. However, there is no effective treatment method until today. Clearance of Aβ and suppression of p-tau are being focused in clinical study of AD.

In sporadic and familial human AD brain samples, AD-induced pluripotent stem cell (iPSC) derived neurons, and AD transgenic animal models, mitochondrial quality control disorder and mitochondrial function decline of neurons are exhibited in AD. Accumulation of these dysfunctional mitochondria contributes to an increase of Aβ and p-tau which are the pathological factors of AD. Moreover, an increase of Aβ and p-tau exacerbates dysfunction of mitochondria (Non-Patent Literatures 1-5). Lack of energy and aggregation of Aβ due to mitochondrial dysfunction leads to synaptic toxicities and amnesia (Non-Patent Literatures 3, 6), and an increase of Aβ and p-tau induces axonal transport defect of mitochondria and leads to synaptic starvation, ATP depletion and finally neurodegeneration (Non-Patent Literature 7). It is revealed that, by restoring or strengthening dysfunctional mitochondria (mitochondrial function or mitochondrial quality control) by a pharmaceutical or genetic method, Aβ toxicity, which is the AD lesion, can be decreased and tau hyperphosphorylation can be invalidated (Non-Patent Literature 1).

UBIAD1 is an enzyme that produces menahydroquinone-4 (MKH) from vitamin K₃ hydroquinone and geranylgeranyl pyrophosphate (Chem. 1) (Non-Patent Literatures 8, 9). In AD patients, UBIAD1 expression level of brain is decreased and MKH level is also decreased (Non-Patent Literature 10). It is revealed that UBIAD1 increases the membrane potential of mitochondria depolarized by a membrane potential depolarizer, and it is indicated that it restores mitochondrial dysfunction and facilitates energy production (Non-Patent Literature 11).

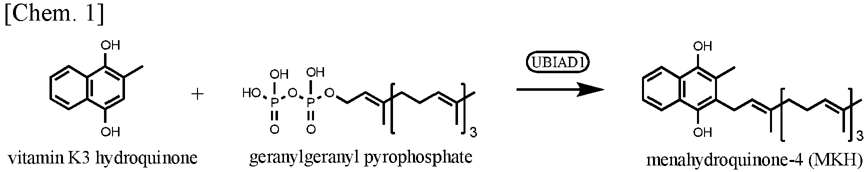

### Biosynthesis of MKH by UBIAD1

Moreover, Parkinson's disease (PD) is a neurodegenerative disease of dopaminergic neurons, and mitochondrial dysfunction and breakdown of mitochondrial quality control cause neuronal death (Non-Patent Literature 12). Protein of PINK1/Parkin is essential in mitophagy related to mitochondrial quality control of PD, and PINK1/Parkin is also included in mitophagy of AD It is reported that UBIAD1 rescues mitochondrial quality control in PD models (Non-Patent Literature 13). Mitochondrial dysfunction of upper motor neurons in amyotrophic lateral sclerosis (ALS) patients is also revealed (Non-Patent Literatures 14, 15).

### Citation List

### Patent Literature

Patent Literature 1: Japanese Patent No. 3088137 B
Patent Literature 2: Japanese Patent No. 4040082 B

### Non-Patent Literature

Non-Patent Literature 1: Fang, E.F., et al., Mitophagy inhibits amyloid-β and tau pathology and reverses cognitive deficits in models of Alzheimer's disease. Nature Neuroscience, 22, 401-412 (2019).
Non-Patent Literature 2: Scheibye-Knudsen, M., et al., Protecting the mitochondrial powerhouse. Trends Cell Biol. 25, 158-170 (2015).
Non-Patent Literature 3: Kerr, J. S. et al. Mitophagy and Alzheimer's disease: cellular and molecular mechanisms. Trends Neurosci. 40, 151-166 (2017).
Non-Patent Literature 4: Selfridge, J. E., et al., Role of mitochondrial homeostasis and dynamics in Alzheimer's disease. Neurobiol. Dis. 51, 3-12 (2013).
Non-Patent Literature 5: Beck, J. S., et al., Evidence for mitochondrial UPR gene activation in familial and sporadic Alzheimer's disease. Curr. Alzheimer Res. 13, 610-614 (2016).
Non-Patent Literature 6: Mairet-Coello, G. et al. The CAMKK2-AMPK kinase pathway mediates the synaptotoxic effects of Aβ oligomers through Tau phosphorylation. Neuron 78, 94-108 (2013).
Non-Patent Literature 7: Vossel, K. A. et al. Tau reduction prevents Aβ-induced defects in axonal transport. Science 330, 198 (2010).
Non-Patent Literature 8: Nakagawa K, et al., Identification of UBIAD1 as a novel human menaquinone-4 biosynthetic enzyme. Nature. 468 (7320) :117-121 (2010).
Non-Patent Literature 9: Hirota Y, et al., Menadione (vitamin K3) is a catabolic product of oral phylloquinone (vitamin K1) in the intestine and a circulating precursor of tissue menaquinone-4 (vitamin K2) in rats. J Biol Chem. 288 (46) :33071-33080 (2013).
Non-Patent Literature 10: Hirota Y, Elucidation of Vitamin K Synthesis Mechanism in Neurodegenerative Diseases, The Uehara Memorial Foundation Reports, 30 (2016) 109.
Non-Patent Literature 11: Fredericks W.J., et al., The TERE1 Protein Interacts with Mitochondrial TBL2: Regulation of Trans-Membrane Potential, ROS/RNS and SXR Target Genes. J. Cell. Biochem., 114, 2170-2187 (2013).
Non-Patent Literature 12: Von Stockum, S., et al., Mitochondrial dynamics and mitophagy in Parkinson's disease: A fly point of view. Neurobiol Dis 90, 58-67 (2016).
Non-Patent Literature 13: Vos M., et al., Vitamin K2 Is a Mitochondrial Electron Carrier That Rescues Pink1 Deficiency. SCIENCE, 336, 1306-1310 (2012).
Non-Patent Literature 14: Gautam, M., et al., Mitoautophagy: A Unique Self-Destructive Path Mitochondria of Upper Motor Neurons With TDP-43 Pathology Take, Very Early in ALS. Front. Cell. Neurosci., 13, 489 (2019).
Non-Patent Literature 15: Gautam, M., et al., Mitochondria, ER, and nuclear membrane defects reveal early mechanisms for upper motor neuron vulnerability with respect to TDP-43 pathology. Acta Neuropathol. 137, 47-69 (2019).

### Summary of Invention

### Technical Problem

From the above background, the inventors hypothesized that by delivering an active form menahydroquinone-4 (MKH) or an active form phyllohydroquinone (PKH) to mitochondria, mitochondrial dysfunction and mitochondrial quality control can be restored and strengthened, and, as a result, the AD lesion (increase of Aβ and p-tau) can be reduced or restored.

The inventors aimed at a low-molecular compound capable of achieving the hypothesis as a drug target, and attempted to search for a candidate compound from prodrugs of MKH and PKH, develop a mitochondrial dysfunction improving agent, prevent AD, and further develop a therapeutic agent, a therapeutic agent for PD, and a therapeutic agent for ALS.

At present, cholinesterase inhibitors and NMDA receptor antagonists are known as therapeutic agents for Alzheimer's disease (AD); however, they merely control AD symptoms, and there is no effective therapy. There is a need for development of an effective and safe therapy that targets at the AD lesion and can change its progress, and suppression of an increase of Aβ and p-tau is focused in clinical study of AD

The present invention was made in view of the prior arts, and the problem to be solved is to provide a vitamin K derivative capable of effectively performing mitochondrial dysfunction and mitochondrial quality control.

### Solution to Problem

As described above, the inventors found that a vitamin K hydroquinone derivative having a specific structure is excellent in deliverability to mitochondria, and thus completed the present invention.

That is, a mitochondrial dysfunction improving agent (vitamin K hydroquinone derivative) according to the present invention comprises a carboxylic acid ester of an active vitamin K represented by the following general formula (1) or a salt thereof.

### General formula (1)

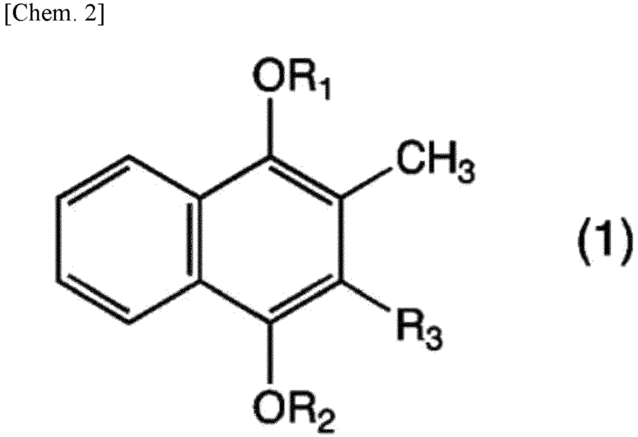

(wherein, R₁ and R₂ are a hydrogen atom, respectively, or a substituent selected from glycine, N-acyl glycine, N-alkyl glycine, N,N-dialkyl glycine, N,N,N-trialkyl glycine, acyl, dicarboxylic acid hemiester, and salts thereof; and at least either of R₁ and R₂ is glycine, N-acyl glycine, N-alkyl glycine, N,N-dialkyl glycine, N,N,N-trialkyl glycine, acyl, dicarboxylic acid hemiester, and salts thereof. R₃ is a group represented by the following general formula (2) 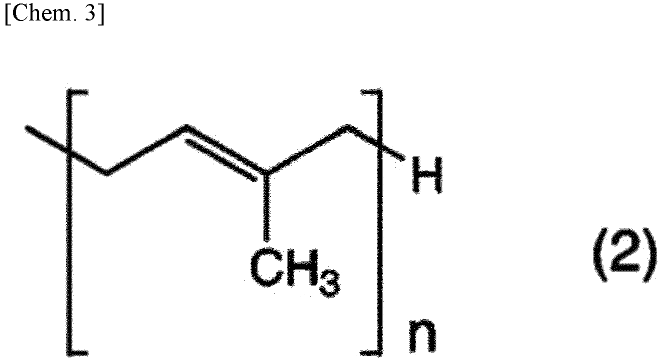 or the following general formula (3), and 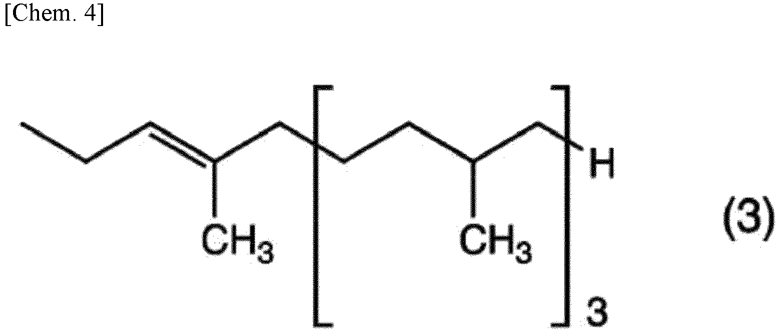 n is an integer of 1 to 7).

Moreover, the vitamin K hydroquinone dicarboxylic acid double ester according to the present invention is represented by the following general formula (4). 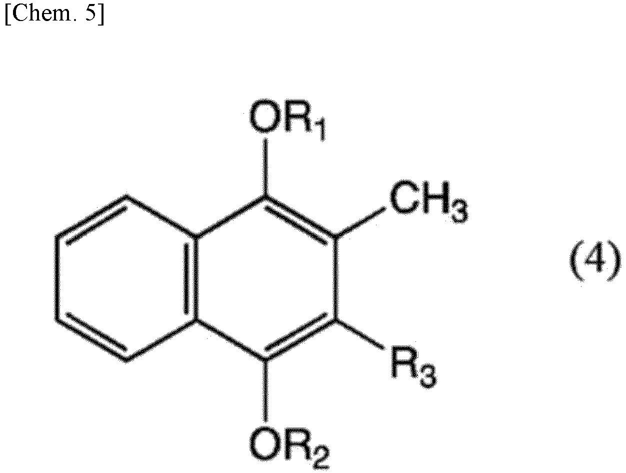 (in the general formula (4), R₁ and R₂ is a carboxylic acid residue selected from a group consisting of R₄OOCCH₂CH₂CO- and R₄OOCCH₂CH₂CH₂CO-. R₃ represents the above general formula (2) or (3). R₄ is a C1-C3 alkyl group.)

The present invention is a mitochondrial dysfunction improving agent having at least one type of a carboxylic acid ester of vitamin K hydroquinone or a salt thereof, or vitamin K hydroquinone dicarboxylic acid double ester represented by the above general formula (1) or (4) as an active ingredient. The mitochondrial dysfunction improving agent effectively delivers an active vitamin K to mitochondria, and is effective in preventing and treating neurodegenerative diseases, such as Alzheimer's disease, Parkinson's disease, and amyotrophic lateral sclerosis, and type-2 diabetes.

The compound represented by the general formula (1) can be added solely into the formulation, or can be formulated into the formulation as its salt. The compound represented by the general formula (4) can be added solely into the formulation. In the present invention, examples of carboxylic acid residues R₁, R₂ having a nitrogen substituent include the following.

Those having a hydrogen atom or 1 or 2 alkyl group or acyl group bonded to the nitrogen atom.

The alkyl group is a linear or branched alkyl group having 1 to 6 carbon atoms, and examples thereof include: methyl group, ethyl group, n-propyl group, n-butyl group, n-pentyl group, n-hexyl group, isopropyl group, isobutyl group, 1-methyl propyl group, tert-butyl group, 1-ethyl propyl group, and isoamyl group. The alkyl group is preferably a methyl group or an ethyl group. Moreover, a hydrocarbon chain of when the acyl group is comprised can be defined similarly.

The amino group and the carbonyl group are preferably bonded with a linear, branched or cyclic alkylene group having 1 to 7 carbon atoms. Examples of the branched alkylene group include those induced from an alkyl group such as isopropyl, isobutyl, tert-butyl, and 1-ethylpropyl.

Examples of the cyclic alkylene group include those comprising a cyclopentane ring, a cyclohexane ring, or a methylcyclohexane ring in its structure. A methylene group or an ethylene group is particularly preferred as the alkylene group.

Hydrochloride and hydrobromide are preferred as a hydrohalic acid salt. In the present invention, the hydrohalic acid salt is advantageous in the point that it crystalizes or solidifies often, and thus handling upon formulation becomes easy. Examples of other salts include: methanesulfonates as alkylsulfonate salts; and gluconates, glucoheptates, and lactobionates as saccharates.

In the present invention, dicarboxylic acid residues R₁, R₂ are selected from dicarboxylic acid and its alkaline metal salt or a residue of a meglumine salt. The carbonyl groups of the dicarboxylic acid residue are bonded with a linear alkylene group having 2 to 4 carbon atoms. An ethylene group or a propylene group is particularly preferred as the alkylene group. A sodium salt or a calcium salt is preferred as the alkaline metal salt.

In the present invention, the carbonyl groups of the dicarboxylic acid residue of the dicarboxylic acid double ester are bonded with a linear alkylene group having 2 to 4 carbon atoms. An ethylene group or a propylene group is particularly preferred as the alkylene group. An alcohol residue of the double ester is preferably an alkyl group having 1 to 3 carbon atoms, and an ethyl group is particularly preferred.

Moreover, in the present invention, there are various methods for producing the compounds represented by the general formula (1) and the general formula (4), and a representative method is as follows.

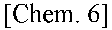

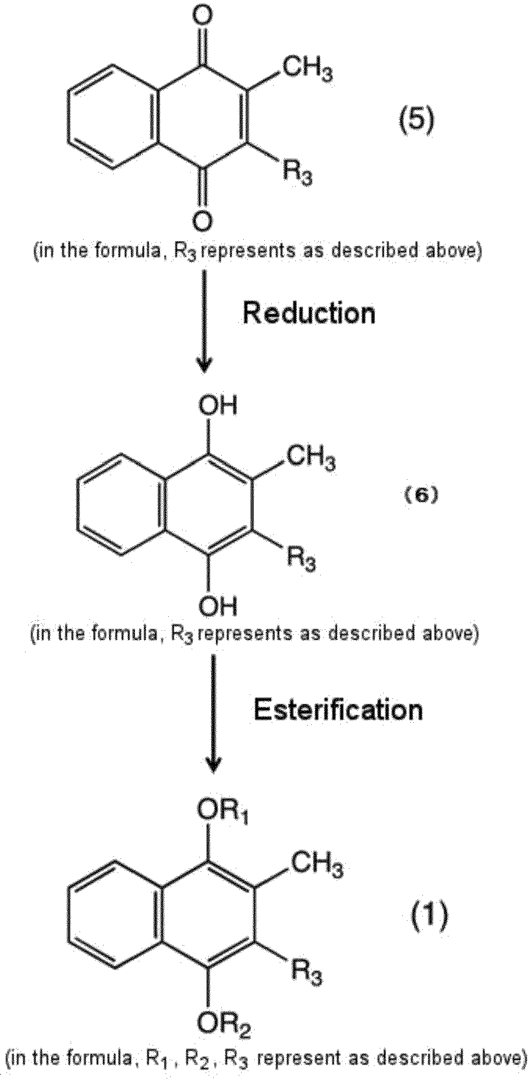

Vitamin K represented by the general formula (5) is reduced with a reducing agent to give a vitamin K hydroquinone represented by the general formula (6). This vitamin K hydroquinone is esterified with a carboxylic acid having a nitrogen substituent, a reactive acid derivative thereof, a hydrohalic acid salt thereof, or an acid anhydride by a conventional method, so that a target substance (1) of the present invention can be obtained. The reducing agent used here reduces a naphthoquinone skeleton of vitamin K to a naphtohydroquinone skeleton, and examples thereof include: sodium borohydride, sodium hydrosulfite, tri-n-butyl phosphine, zinc chloride, stannous chloride, and zinc powders.

Esterification of vitamin K hydroquinone is carried out by a conventional method. Upon esterification of an amino acid having a primary or secondary amino group or having a hydroxyl group or a thiol group in the side chain, it is preferred to protect these primary or secondary amino groups, hydroxyl group, and thiol group with a suitable protecting group such as tert-butoxycarbonyl group (hereinafter abbreviated as t-BOC group), benzyloxycarbonyl group (hereinafter abbreviated as Z group), and 9-fluorenylmethyloxycarbonyl group (hereinafter abbreviated as FMOC group). As for N,N-dialkylamino acid, a preferred result can be achieved by using a hydrohalic acid salt and carrying out the reaction in the presence of an active esterification reagent such as dicyclohexylcarbodiimide (hereinafter abbreviated as DCC) and N,N-disuccinimidyl oxalate (hereinafter referred to as DSO). Here, anhydrous pyridine is preferred as a solvent. Moreover, in the method using the reactive acid derivative, a method using an acid halogenide, especially an acid chloride, achieves a preferred result. Here, a mixture of anhydrous benzene-anhydrous pyridine is preferred as a solvent. Hydrohalic acid salts, alkylsulfonate salts, acidic sugar salts may be produced, according to a conventional method, by the reaction of a free vitamin K hydroquinone nitrogen containing carboxylic acid ester and lactones of a hydrohalic acid salt, alkylsulfonate salt, and saccharate. In addition, after the production of an N-acyl amino acid ester, hydrohalic acid salts can be produced by deprotection with a hydrohalic acid by a conventional method.

As for the dicarboxylic acid double ester represented by the general formula (4), vitamin K represented by the general formula (5) is reacted by heating with zinc powder and acid anhydride under acetic-acid acidity condition to give a vitamin K hydroquinone carboxylic acid hemiester, and further esterified with an alcohol in the presence of an acidic catalyst to obtain the target substance (4). Moreover, the vitamin K hydroquinone carboxylic acid hemiester obtained by the general formula (1) is esterified with an alcohol in the presence of an acidic catalyst to obtain the target substance (4).

The vitamin K hydroquinone derivative of the present invention has a high degree of freedom of dosage forms, and oral administration, dermal administration, intranasal administration, or administration by injection can be adopted. Isotonic agents, buffers, pH adjusters, solubilizers, thickeners (dispersants), mucoadhesive agents, stabilizers (antioxidants), preservatives (antiseptics), and absorption promoters may be added suitably as an additive, so that it can be formulated by a well-known method. Moreover, by adding pH adjusters, thickeners and dispersants to suspend the drug, stable eye drops or nasal drops can be obtained.

Isotonic agents are not limited in particular so long as they are pharmacologically (pharmaceutically) or physiologically acceptable in terms of medicine. Examples ionic isotonic agents include sodium chloride, potassium chloride, calcium chloride, and magnesium chloride. Examples of non-ionic isotonic agents include glycerin, propylene glycol, sorbitol, and mannitol.

Buffers are not limited in particular so long as they are pharmacologically (pharmaceutically) or physiologically acceptable in terms of medicine. Examples thereof include phosphoric acid, phosphate, citric acid, acetic acid or ε-aminocaproic acid.

pH adjusters are not limited in particular so long as they are pharmacologically (pharmaceutically) or physiologically acceptable in terms of medicine. Examples thereof include hydrochloric acid, phosphoric acid, citric acid, acetic acid, sodium hydroxide, potassium hydroxide, boric acid, borax, sodium carbonate, and sodium hydrogen carbonate. pH of the eye drops may be within a range acceptable for ophthalmological formulations, and may be within a range of 4.0 to 9.0, more preferably 5.5 to 8.5.

Solubilizers are not limited in particular so long as they are pharmacologically (pharmaceutically) or physiologically acceptable in terms of medicine. Examples thereof include polyoxyethylene castor oil, polyoxyethylene hydrogenated castor oil, polyoxyethylene sorbitan fatty acid esters, vitamin E TPGS, polyoxyethylene fatty acid esters, polyoxyethylene polyoxypropylene glycol, and sucrose fatty acid esters.

Thickeners, dispersants and mucoadhesive agents are not limited in particular so long as they are pharmacologically (pharmaceutically) or physiologically acceptable in terms of medicine. Examples thereof include: cellulose-based polymers such as hydroxypropyl methyl cellulose or hydroxypropyl cellulose; polyvinyl alcohol; or polyvinylpyrrolidone.

Stabilizers are not limited in particular so long as they are pharmacologically (pharmaceutically) or physiologically acceptable in terms of medicine. Examples thereof include edetic acid, monosodium edetate, disodium edetate, tetrasodium edetate, and sodium citrate. Sodium edetate may be a hydrate.

Antioxidants are not limited in particular so long as they are pharmacologically (pharmaceutically) or physiologically acceptable in terms of medicine. Examples thereof include ascorbic acid, vitamin E, dibutyl hydroxy toluene, butylated hydroxyanisole, sodium erythorbate, propyl gallate, and sodium sulfite.

Preservatives (antiseptics) are not limited in particular so long as they are pharmacologically (pharmaceutically) or physiologically acceptable in terms of medicine. Examples thereof include benzalkonium chloride, benzalkonium bromide, benzethonium chloride, sorbic acid, potassium sorbate, methyl parahydroxybenzoate, propyl parahydroxybenzoate, and chlorobutanol, and these preservatives can be used in combination.

### Advantageous Effects of Invention

As described above, the mitochondrial dysfunction improving agent according to the present invention enables delivery of an active vitamin K to mitochondria, and can improve various diseases caused by mitochondrial dysfunction.

### Brief Description of Drawings

Fig. 1 illustrates the improvement effect by an MKH derivative to a neurological disorder caused by β-amyloid.
Fig. 2A illustrates the improvement effect by an MKH derivative to a neurological disorder caused by β-amyloid.
Fig. 2B illustrates the improvement effect by an MKH derivative to a neurological disorder caused by β-amyloid.
Fig. 3A illustrates the improvement effect by an MKH derivative to a neurological disorder caused by β-amyloid.
Fig. 3B illustrates the improvement effect by an MKH derivative to a neurological disorder caused by β-amyloid.
Fig. 4 illustrates the improvement effect of cell death by an MKH derivative. vs control (** = p < 0.01), vs Rot (## = p < 0.01)
Fig. 5 illustrates the improvement effect of suppression of ATP production by an MKH derivative. vs control (** = p < 0.01), vs Rot (## = p < 0.01)
Fig. 6A illustrates the recovery state of the membrane potential by an MKH derivative.
Fig. 6B illustrates the recovery state of the membrane potential by an MKH derivative. vs control (** = p < 0.01), vs Rot (## = p < 0.01)
Fig. 7 illustrates the improvement effect of cell death by a PKH derivative. vs control (** = p < 0.01), vs Rot (## = p < 0.01)
Fig. 8 illustrates the improvement effect of suppression of ATP production by a PKH derivative. vs control (** = p < 0.01), vs Rot (## = p < 0.01)
Fig. 9 illustrates the recovery effect of the membrane potential by a PKH derivative. vs control (** = p < 0.01), vs Rot (## = p < 0.01)
Fig. 10 illustrates the recovery effect of the membrane potential to Complex II inhibitor by an MKH derivative. vs control (** = p < 0.01), vs 3-NP (## = p < 0.01)
Fig. 11 illustrates the recovery effect of the membrane potential to Complex III inhibitor by an MKH derivative. vs control (** = p < 0.01), vs antimycin A (## = p < 0.01)
Fig. 12 illustrates the effect of an MKH derivative to cell death caused by an uncoupler (carbonylcyanide-m-chlorophenylhydrazone, CCCP). vs control (** = p < 0.01), vs CCCP (## = p < 0.01)

### Description of Embodiments

In the following, preferred embodiments of the present invention are described in detail.

In sporadic and familial human AD brain samples, AD-induced pluripotent stem cell (iPSC) derived neurons, and AD transgenic animal models, mitochondrial quality control disorder and mitochondrial function decline of neurons are exhibited in AD, and it was revealed that accumulation of these dysfunctional mitochondria contributes to an increase of Aβ and p-tau which are the factors of the AD lesion (Non-Patent Literatures 1-5). It was revealed that Aβ toxicity can be decreased and tau hyperphosphorylation can be invalidated by restoring or strengthening dysfunctional mitochondria (mitochondrial functions or mitochondrial quality control) with a pharmaceutical or genetic method (Non-Patent Literature 1). It is shown that UBIAD1 is an enzyme that biosynthesizes menahydroquinone-4 (MKH) (Chem. 1), UBIAD1 expression of the brain is decreased in AD patients, MKH level is also decreased, and UBIAD1 increases the membrane potential of polarized mitochondria, i.e., improvement in mitochondrial dysfunction (Non-Patent Literature 13).

Therefore, the inventors hypothesized that mitochondrial dysfunction or mitochondrial quality control can be restored and strengthened, and the AD lesion (Aβ and p-tau) can be reduced or restored by the effective delivery of MKH to mitochondria of cranial nerves of AD patients, and aimed to develop a low-molecular compound capable of MKH, PKH delivery and reducing or restoring the AD lesion (increase of Aβ and p-tau) as a drug target.

MKH is a two-electron reductant of vitamin K₂₍₂₀₎ (menaquinone-4, MK-4) which is a type of vitamin K(VK). In an endoplasmic reticulum, MKH functions as a cofactor of an enzyme (GGCX) that carboxylates a Glu residue to a Gla residue in post-translational modification of a vitamin K-dependent protein (VKDP) which is one action of vitamin K as an activator of VK. Since MKH is extremely easily oxidized, a quinone-type MK-4 is used in clinical study; however, low photostability, high phototoxicity, low water solubility, and necessity of a reductive activation process were problems upon drug delivery of MKH by MK-4. The inventors succeeded to produce an MKH delivery agent that does not require a reductive activation process and has a high photostability and no phototoxicity by making MKH a prodrug.

First, a low-molecular compound capable of restoring and strengthening mitochondrial function and decreasing Aβ protein toxicity by the MKH delivery hypothesized in this study was aimed as a drug target, and an MKH prodrug was focused. Its functions were investigated, and possibility as AD prevention and a therapeutic agent was evaluated in vitro.

Moreover, Parkinson's disease (PD) is a neurodegenerative disease of dopaminergic neurons, and disruption of mitochondrial dysfunction and mitochondrial quality control lead to neuronal cell death in PD. Furthermore, it is reported that UBIAD1 rescues mitochondrial quality control in PD models.

Therefore, an MKH prodrug is used as a drug target of a low-molecular compound to investigate whether mitochondrial dysfunction can be restored and evaluate possibility as a therapeutic agent of PD in vitro.

### (1) Possibility as prevention and therapeutic agent for Alzheimer's disease

In a neuronopathy model due to Aβ peptide of which a single neuron-astrocyte coculture sample is treated with Aβ peptide, an improvement effect in neurodegeneration of a MKH derivative was evaluated. Since mitochondrial affinity of the derivative and reconvertibility to MKH are expected affect the MKH delivery by an MKH derivative, a cationic derivative (MKH-DMG) presumed to have a high affinity, an anionic derivative (MKH-SUC) having a high intracellular reconversionability, and an oxidant of MKH (MK-4) used in clinical study were evaluated (Chem. 7).

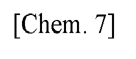

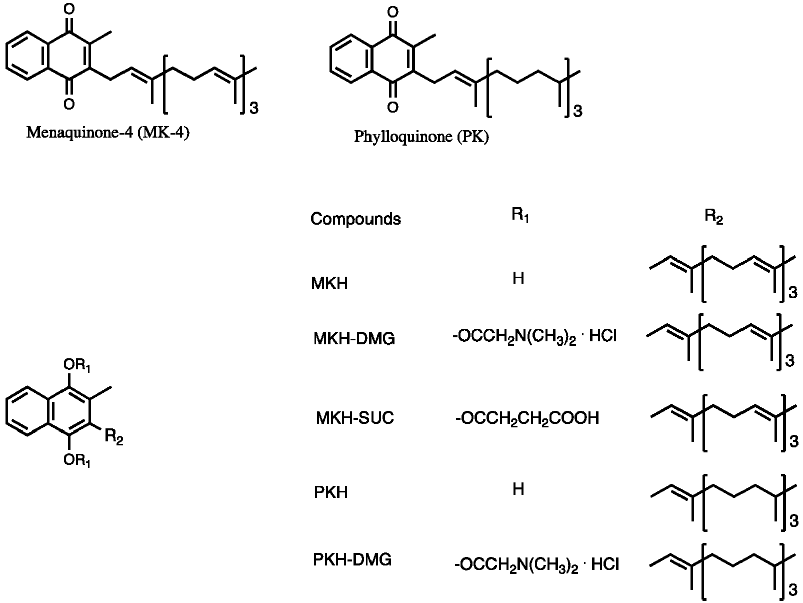

### Quinone-type vitamin K and active (hydroquinone-type) vitamin K derivatives

### Improvement effect of MKH derivative to neuropathy caused by β-amyloid

After culturing (2 weeks) astrocytes isolated from the cerebral cortex of 0- to 1-day-old ICR mouse, regions were divided in dots on a slide glass and cultured. After culturing for one more week, hippocampus-derived neurons of 0- to 1-day-old ICR mouse were seeded and cocultured. From day 1 of seeding neuron, Aβ₂₅₋₃₅ (1 µM) and a test compound (0.3 µM) were exposed for three days, and morphological change of neurons was observed. Dendrites (MAP2 antibody) and axons (Tau antibody) of nerves were stained by immunostaining and analyzed. Dendrites and axons were evaluated for changes in branches and elongation of dendrites and axons by Sholl analysis method.

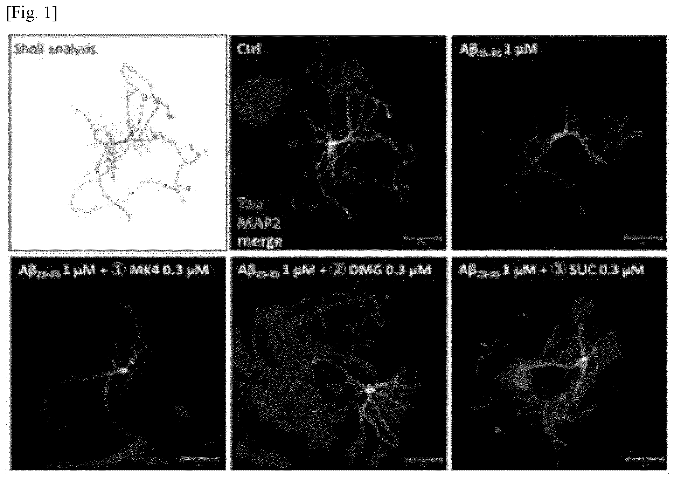

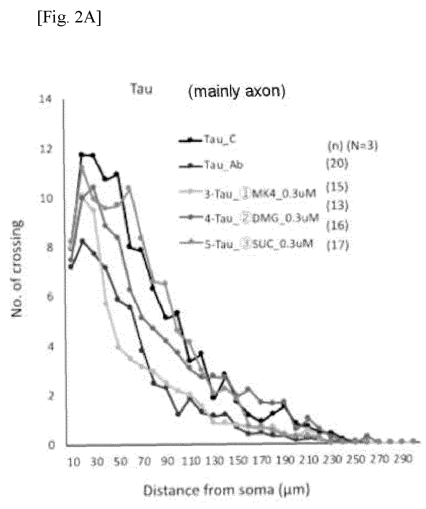

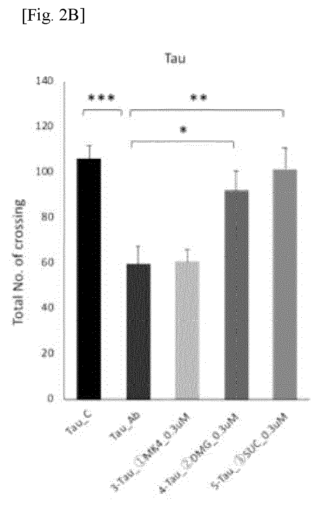

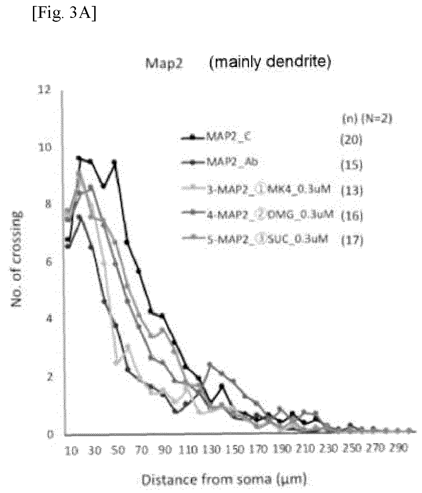

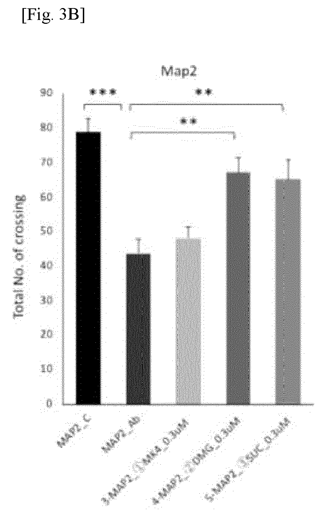

Results of staining of neurons are shown in Fig. 1. Both of branches and elongation of nerve axons and branches and elongation of nerve dendrites were significantly suppressed by Aβ₂₅₋₃₅, and neuropathy was observed. In MKH derivatives (DMG(MKH-DMG) and SUC(MKH-SUC)), both neuropathies of nerve axons and nerve dendrites were significantly improved (Figs. 2 and 3), and the improvement effect of neuropathy was revealed. No improvement effect was observed for MK4 (MK-4) to both neuropathies. Since MKH derivatives have the improvement effect to β-amyloid at a low concentration of 0.3 µM, a high safety can be expected.

### 2) Improvement effect of mitochondrial dysfunction by MKH derivative

Effects of an MKH derivative and a PKH derivative to mitochondrial dysfunctions caused by Complex I inhibitor rotenone (10 µM), Complex II inhibitor (3-nitro propionic acid (3-NP)), Complex III inhibitor (antimycin A), and a depolarizing agent (carbonylcyanide-m-chlorophenylhydrazone, CCCP) in neural cells were evaluated. The effects were evaluated with cell death (viability measurement with cell titer blue), inhibition of ATP production (ATP measurement with cell titer glo), and membrane potential reduction (JC-1 staining: depolarization (green), hyperpolarization (red)).

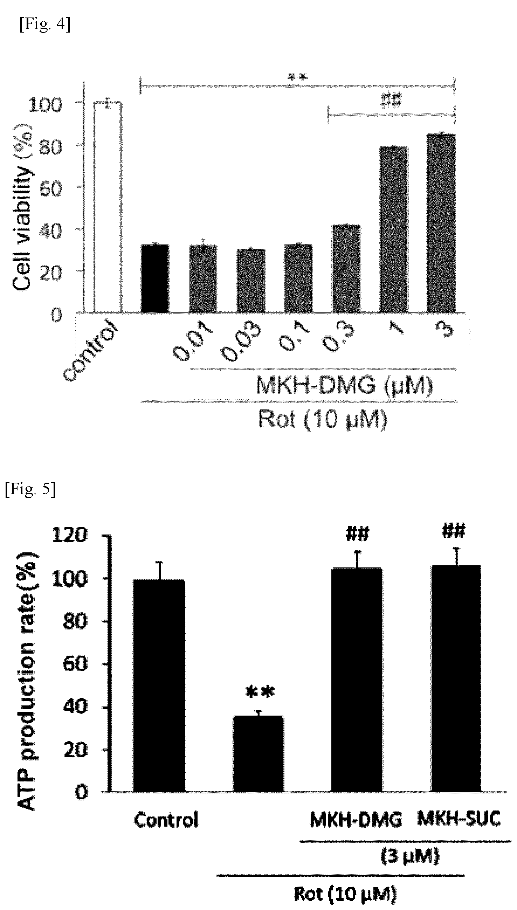

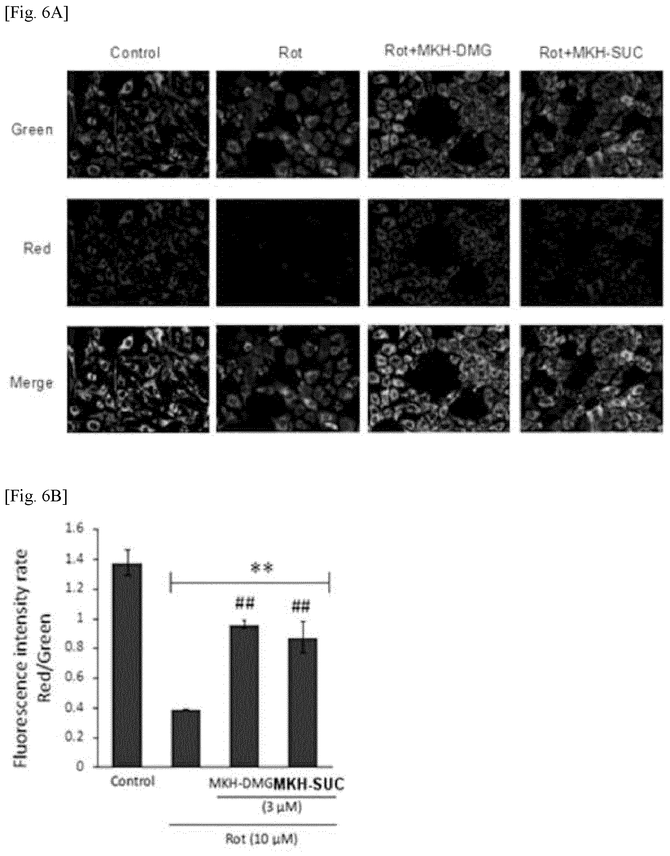

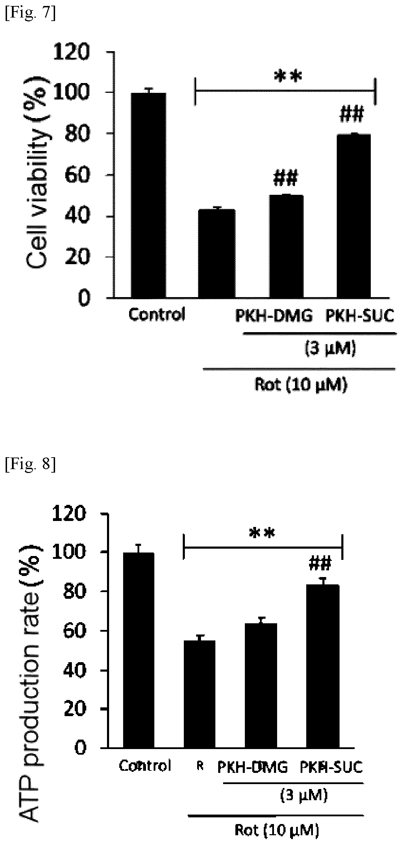

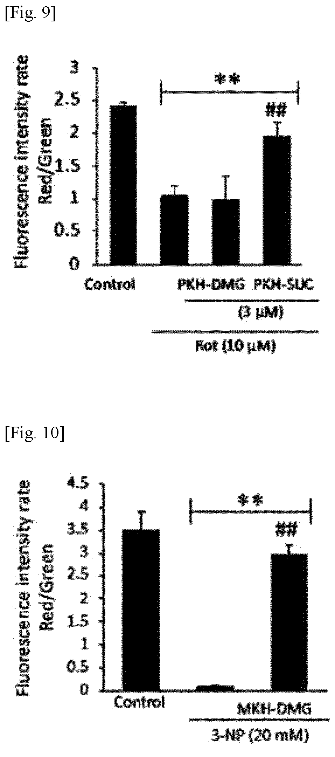

By MKH derivative administration, cell death caused by Complex I inhibitor rotenone treatment was suppressed (Fig. 4), and inhibition of ATP production (Fig. 5) and mitochondrial membrane potential reduction were restored (Fig. 6). In PKH derivative administration, cell death caused by rotenone treatment was suppressed (Fig. 7), and inhibition of ATP production (Fig. 8) and mitochondrial membrane potential reduction were restored (Fig. 9).

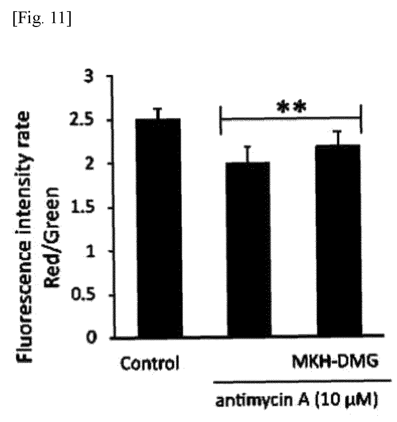

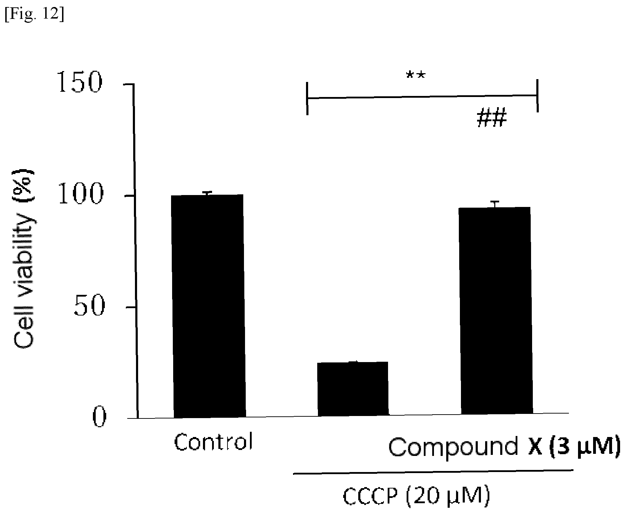

Moreover, each derivative restored membrane potential reduction (Fig. 10). Furthermore, each derivative restored reduction in viability, ATP production and membrane potential caused by Complex III inhibitor (antimycin A) (Fig. 11). In addition, each derivative restored reduction in viability caused by a depolarization agent (CCCP) (Fig. 12). Moreover, each derivative increased expression of PGC-1α (peroxisome proliferator-activated receptor-c coactivator-1α) that activates mitochondrial biogenesis.

From the above results, it was revealed that the MKH derivative and PKH derivative can restore mitochondrial dysfunction (membrane potential reduced mitochondria).

### 3) Possibility as PD therapeutic agent

Complex I activity is decreased in sporadic PD. In rotenone Complex I inhibitor models, morphological and functional damages are caused in dopaminergic neurons in addition to characteristic symptoms of PD such as bradykinesia, rigidity, and tremor. Moreover, since mitochondria damaged by Complex I inhibition (membrane potential reduced mitochondria) induces mitochondrial quality control, it is used for evaluation of hereditary PD. Therefore, the acquired results revealed that the MKH derivative and PKH derivative are effective in rotenone PD models, and possibility as a PD therapeutic agent was revealed.

From the above evaluation results:
- it was revealed that, by using primary neurons, MKH derivatives of MKH-DMG and MKH-SUC can improve neurotoxicity caused by Aβ at a low concentration of 0.3 µM; and
- it was revealed that MKH-DMG and MKH-SUC can improve mitochondrial function (respiratory chain) damage.

These results support the hypothesis that mitochondrial dysfunction or mitochondrial quality control can be restored or strengthened and the AD lesion (increase of Aβ and p-tau) can be reduced or restored by the effective delivery of MKH to cranial nerve mitochondria of AD patients, and it was shown that the MKH derivative have a high possibility of a drug target based on the hypothesis.

Moreover, the effect of the MKH derivative in rotenone PD models supports a possibility as a PD therapeutic agent by restoration and strengthening of mitochondrial quality control.

Moreover, it supports a possibility as a therapeutic agent for amyotrophic lateral sclerosis by mitochondrial function restoration.

Therefore, the inventors propose an MKH derivative as a drug target of a preventative/therapeutic agent for AD and a therapeutic agent for PD having restoration and strengthening of mitochondrial dysfunction or quality control as a mechanism of action.

The inventors hypothesize that the AD lesion (increase of Aβ and p-tau) can be decreased by enabling restoration and strengthening of mitochondrial dysfunction or mitochondrial quality control by the effective delivery of MKH to mitochondria of cranial nerves of AD patients, and aim to develop a low molecular compound that enables the MKH delivery and a reduction of the AD lesion (increase of Aβ and p-tau) as a drug target. So far, the inventors evaluated an original MKH prodrug, and succeeded to reveal a high possibility.

Originality: A reduction of the AD lesion (increase of Aβ and p-tau) by restoration and strengthening of mitochondrial dysfunction or mitochondrial quality control has been shown in AD model cells or AD model mice; however, there is no method using the MKH delivery by an original MHK prodrug, and this method is the first method in the world.

High safety: Since improvement of neurotoxicity caused by Aβ using an original MHK prodrug is effective at a drug concentration of 0.3 µM, the effect can be exhibited at a low concentration. An MKH prodrug is a low molecular compound, and it is expected that after functioning as an MKH, it follows the same fate as vitamin K and disappears. Therefore, an MKH prodrug is considered to be a highly safe prevention/therapeutic agent with extremely low risk for harmful side effects.

### Examples

The present invention is described in further details with reference to Examples in the following; however, the present invention is not limited thereto.

### Examples 1 to 34

Vitamin K hydroquinone derivatives shown in Tables 1 to 5 are produced by the methods A to I in the following. Moreover, mass spectra (ionizing method; FD method and FAB method) and ¹H-NMR spectra of the obtained substances are shown in Tables 6 to 8.

### Production method A

Amino acid (0.1 mol) is dissolved in 100 ml of distilled water-dioxane (1:1, v/v), and 30 ml of triethylamine is added thereto. Then, di-tert-butyl decarbonate is gradually added thereto, and the mixture is stirred for 30 minutes at room temperature. Dioxane is removed under reduced pressure. 50 ml of sodium hydrogen carbonate aqueous solution (0.5 M) is added thereto, and washed with 100 ml of ethyl acetate. The ethyl acetate layer is washed with 50 ml of sodium hydrogen carbonate solution. The aqueous layer is combined and adjusted to be acidic (pH 3) with a citric acid aqueous solution (0.5 M) under cooling with ice, saturated with sodium chloride, and then extracted with ethyl acetate (100 ml x 3 times). The extract is dehydrated with anhydrous sodium sulfate, and the solvent is removed under reduced pressure. A N-t-BOC-amino acid is obtained by adding isopropyl ether to the oily residue or by the crystallization thereof under cooling. Vitamin K (6.75 mmol) is dissolved in 40 ml of isopropyl ether, and sodium borohydride (47 mmol) is dissolved in 15 ml of methanol and added thereto. The mixture is stirred at room temperature until the color of the solution changes from yellow to colorless. 60 ml of isopropyl ether and 100 ml of distilled water are added to the reaction solution, and the isopropyl ether layer is separated. 100 ml of isopropyl ether is further added to the aqueous phase to extract a soluble fraction. The isopropyl layer is combined, and the mixture is dehydrated with anhydrous sodium sulfate and then is concentrated under reduced pressure. n-Hexane is added to the residue and a white precipitate is deposited to obtain vitamin K hydroquinone.

Vitamin K hydroquinone, N-t-BOC-amino acid (13.55 mmol) and DCC (13.55 mmol) are added to 50 ml of anhydrous pyridine, and the mixture is stirred for 20 hours at room temperature. The solvent is removed under reduced pressure. Ethyl acetate is added to the residue to extract a soluble fraction (100 ml x 2 times). The extract is concentrated under reduced pressure, and the residue is separated and purified by silica gel column chromatography (elution solvent: n-hexane-isopropyl ether) to obtain vitamin K hydroquinone-1,4-bis-N-t-BOC-amino acid. Vitamin K hydroquinone-1,4-bis-N-t-BOC-amino acid is dissolved in a small amount of acetone. Hydrochloric acid-dioxane (2.5-4.0 N) is added thereto so that the amount of hydrochloric acid is about 20 times of the ester in moles. After stirring for 1 hour, the solvent is removed under reduced pressure. The residue is recrystallized with acetone-methanol system to obtain a hydrochloride of vitamin K hydroquinone-1,4-bis-amino acid aster.

### Production method B

Vitamin K (6.75 mmol) is dissolved in 40 ml of isopropyl ether, and sodium borohydride (47 mmol) is dissolved in 15 ml of methanol and added thereto. The solution is stirred at room temperature until the color of the solution changes from yellow to colorless. 60 ml of isopropyl ether and 100 ml of distilled water are added to the reaction solution, and the isopropyl ether layer is separated. 100 ml isopropyl ether is further added to the aqueous phase to extract a soluble fraction, and the isopropyl layer is combined. After dehydrating with anhydrous sodium sulfate, the extract is concentrated under reduced pressure. n-Hexane is added to the residue and a white precipitated is deposited to obtain vitamin K hydroquinone. Vitamin K hydroquinone, N,N-dialkylamino acid hydrochloride (13.55 mmol) or N,N,N-trialkylamino acid hydrochloride (13.55 mmol), and DCC (13.55 mmol) are added to 50 ml of anhydrous pyridine, and the mixture is stirred for 20 hours at room temperature. The solvent is removed under reduced pressure, and the residue is suspended in distilled water, adjusted to pH 7 to 8 with sodium hydrogen carbonate, and then extracted with ethyl acetate (100 ml x 3 times). After dehydrating the extract with anhydrous sodium sulfate, the solvent is removed under reduced pressure. The residue is separated and purified by silica gel column chromatography (elution solvent: isopropyl ether-ethyl acetate) to obtain vitamin K hydroquinone-1,4-bis-N,N-dialkylamino acid ester or vitamin K hydroquinone-1,4-bis-N,N,N-trialkylamino acid aster.

### Production method C

Vitamin K (6.75 mmol) is dissolved in 40 ml of isopropyl ether, and hydrosulfite sodium (50 mmol) is dissolved in 50 ml of distilled water and added thereto. The mixture is stirred at room temperature until isopropyl ether exhibits brown color and then further changes to colorless. The isopropyl layer is separated, and 100 ml of isopropyl ether is further added to the aqueous phase to extract a soluble fraction. The isopropyl layer is combined, and after dehydrating with anhydrous sodium sulfate, the extract is concentrated under reduced pressure. n-Hexane is added to the residue and a white precipitate is deposited to obtain vitamin K hydroquinone. N,N-dialkylamino acid hydrochloride (6.75 mmol) and DCC (6.75 mmol) are added to vitamin K hydroquinone, and the mixture is stirred in 50 ml of anhydrous pyridine for 20 hours. The solvent is removed under reduced pressure, and the residue is suspended in distilled water, adjusted to pH 7 to 8 with sodium hydrogen carbonate, and then extracted with ethyl acetate (100 ml x 3 times). After the extract is dehydrated with anhydrous sodium sulfate, the solvent is removed. The residue is separated and purified by silica gel column chromatography (elution solvent: isopropyl ether-ethyl acetate, 3:2) to obtain vitamin K hydroquinone-1-N,N-dialkylamino acid ester and vitamin K hydroquinone-4-N,N,-dialkylamino acid ester.

### Production method D

Vitamin K (6.75 mmol) is dissolved in 40 ml of isopropyl ether, and sodium borohydride (47 mmol) is dissolved in 15 ml of methanol and added thereto. The solution is stirred until the color of the solution changes from yellow to colorless at room temperature. 60 ml of isopropyl ether and 100 ml of distilled water is added to the reaction solution, and the isopropyl ether layer is separated. 100 ml of isopropyl ether is further added to the aqueous layer to extract a soluble fraction, and the isopropyl ether layer is combined. After dehydrating with anhydrous sodium sulfate, the extract is concentrated under reduced pressure. n-Hexane is added to the residue and a white precipitate is deposited to obtain vitamin K hydroquinone. Vitamin K hydroquinone is dissolved in 30 ml of anhydrous benzene-anhydrous pyridine (1:1, v/v), pyridinecarbonyl chloride hydrochloride is added thereto, and stirred for 3 hours at room temperature. Insoluble matters are removed by filtration, and the filtrate is concentrated under reduced pressure. The residue is suspended in 100 ml of distilled water, and sodium hydrogen carbonate is added thereto (pH 7-8) to extract a soluble fraction to ethyl acetate (100 ml x 3 times). The extract is concentrated under reduce pressure. The residue is separated and purified by silica gel column chromatography (elution solvent: isopropyl ether-ethyl acetate, 9:1) to obtain vitamin K hydroquinone-1,4-bis-pyridinecarboxylic acid ester.

### Production method E

Vitamin K hydroquinone-1,4-bis-N,N-dialkylamino acid ester or vitamin K hydroquinone-1,4-bis-pyridinedicarboxylic acid (2 mmol) is dissolved in 20 ml of acetone, and hydrochloric acid-dioxane (2.5-4.0 N) is added thereto so that the amount of hydrochloric acid is 10 times of the ester in moles. The solvent is removed under reduced pressure. The residue is recrystallized with acetone-methanol to obtain a hydrochloride of vitamin K hydroquinone-1,4-bis-N,N-dialkylamino acid or vitamin K hydroquinone-1,4-bis-pyridinecarboxylic acid.

### Production method F

Vitamin K hydroquinone-1,4-bis-N,N-dialkylamino acid or vitamin K hydroquinone-1,4-bis-pyridinedicarboxylic acid (2 mmol) is dissolved in 20 ml of dichloromethane. Alkyl sulfonic acid (2 mmol) is added thereto and stirred. Depositing crystals are filtered to obtain an alkylsulfonate salt of vitamin K hydroquinone-1,4-bis-N,N-dialkylamino acid ester or vitamin K hydroquinone-1,4-bis-pyridinedicarboxylic acid ester.

### Production method G

Vitamin K (4.55 mmol) is dissolved in 40 ml of isopropyl ether, and sodium borohydride (31.5 mmol) is dissolved in 15 ml of methanol and added thereto. The solution is stirred until the color of the solution changes from yellow to colorless at room temperature. 60 ml of isopropyl ether and 100 ml of purified water are added to the reaction solution, and the isopropyl ether layer is separated. 100 ml of isopropyl ether is further added to the aqueous phase to extract a soluble fraction, and the isopropyl layer is combined. After dehydrating with anhydrous sodium sulfate, the solvent is removed under reduced pressure. Dimethylaminopyridine (8.97 mmol) and dicarboxylic anhydride (18.0 mmol) are added to the residue, dissolved in 100 ml of isopropyl ether-dioxane (6:4, v/v), and stirred for 3 hours at room temperature. Then, the solution is heated to 50-60 °C to react for 2 hours, and allowed to cool at room temperature to react for 10 hours. 100 ml of purified water is added to the reaction solution, and the isopropyl ether layer is separated. After dehydrating with anhydrous sodium sulfate, the solvent is removed under reduced pressure. The residue is suspended in isopropyl ether, and centrifuged to obtain a precipitate. 100 ml of ethyl acetate and 100 ml of purified water are added to the precipitate to extract an ethyl acetate soluble fraction. After dehydrating with anhydrous sodium sulfate, the solvent is removed under reduced pressure. The residue is suspended in isopropyl ether and insoluble matters are recrystallized with ethyl acetate to obtain a vitamin K hydroquinone-1,4-bis-dicarboxylic acid hemiester.

### Production method H

Vitamin K (6.75 mmol), zinc (18.4 mmol), anhydrous dicarboxylic acid (33.0 mmol), anhydrous sodium acetate (13.8 mmol), and acetic acid (161.5 mmol) are put into a 100 ml eggplant flask, Dimroth condenser is mounted thereto, and the mixture is heated at 85 °C for 3 hours while being stirred well. Then, the mixture is cooled at room temperature to obtain a white solid substance. 200 ml of ethyl acetate and 100 ml of purified water are added thereto to extract an ethyl acetate soluble fraction. After dehydrating with anhydrous sodium sulfate, the solvent is removed under reduced pressure. The residue is recrystallized with ethyl acetate to obtain a vitamin K hydroquinone-1,4-bis-dicarboxylic acid hemiester.

### Production method I

Vitamin K hydroquinone-1,4-bis-dicarboxylic acid hemiester (2 mmol) is added to 0.1 N sodium hydroxide aqueous solution (2 times in moles) or meglumine aqueous solution (2 times in moles), and freeze dried. It is recrystallized with methanol-acetonitrile to obtain vitamin K hydroquinone-1,4-bis-dicarboxylic acid hemiester-bis-sodium salt or vitamin K hydroquinoe-1,4-bis-dicarboxylic acid hemiester-bis-meglumine salt.

### Production method J

Vitamin K hydroquinone-1,4-bis-dicarboxylic acid hemiester is dissolved in primary or secondary alcohol, and stirred under hydrochloric acid acidity. The solvent is removed under reduced pressure to obtain vitamin K hydroquinone-1,4-bis-dicarboxylic acid alcohol ester.

### [Table 1]

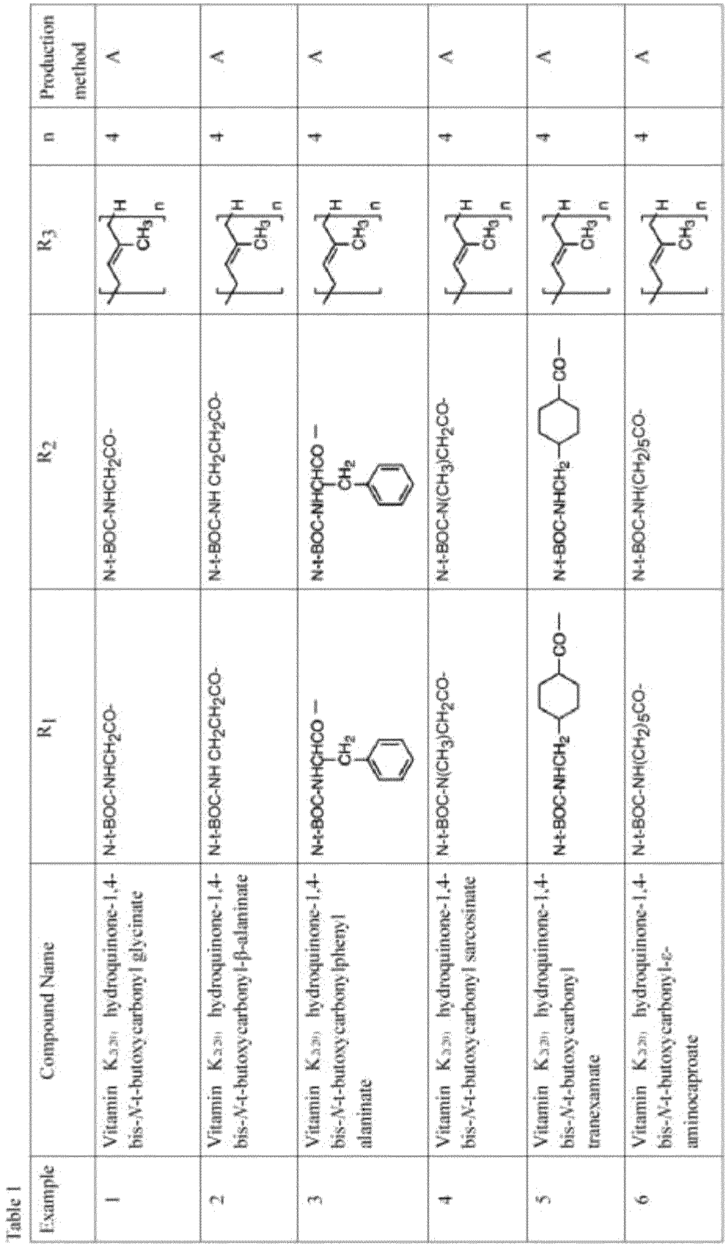

### [Table 2]

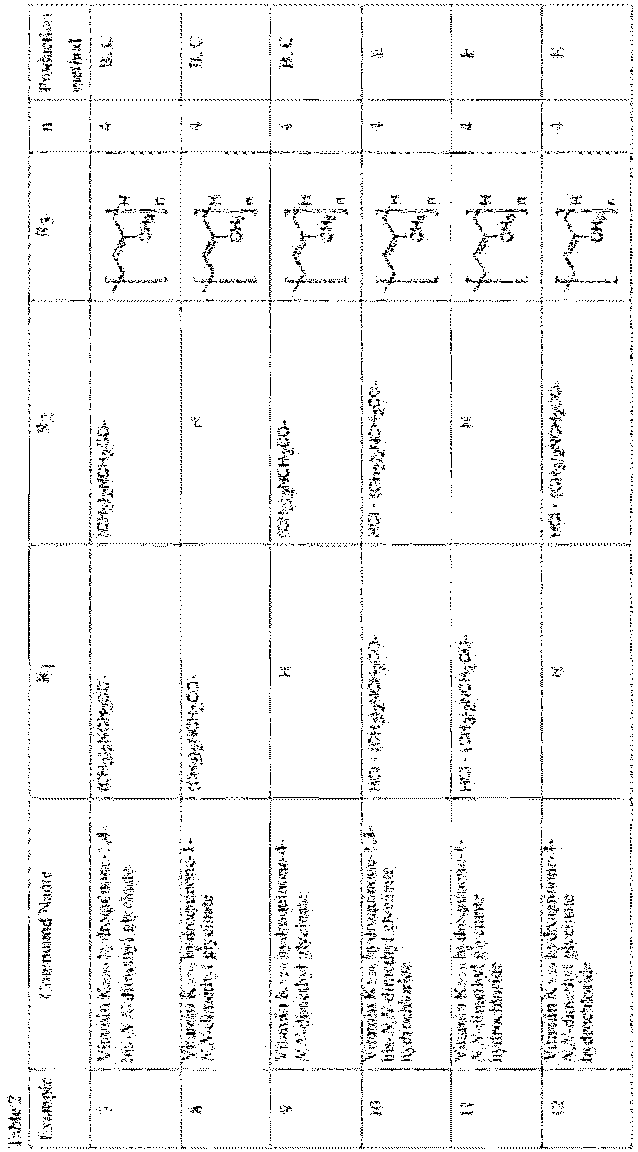

### [Table 3]

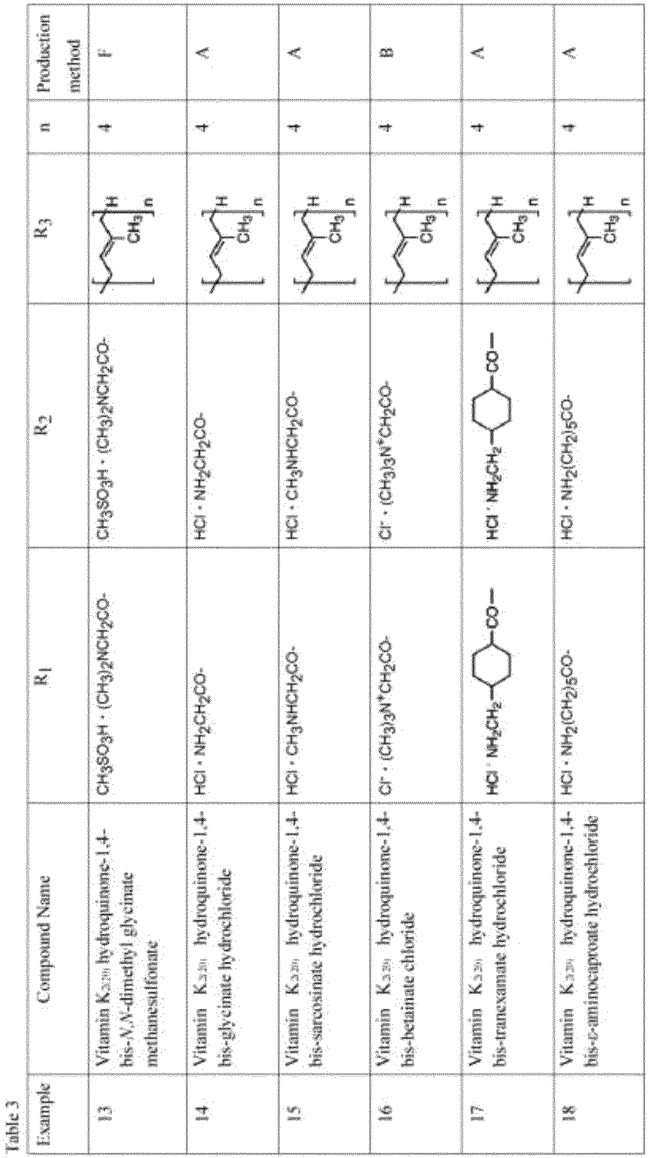

### [Table 4]

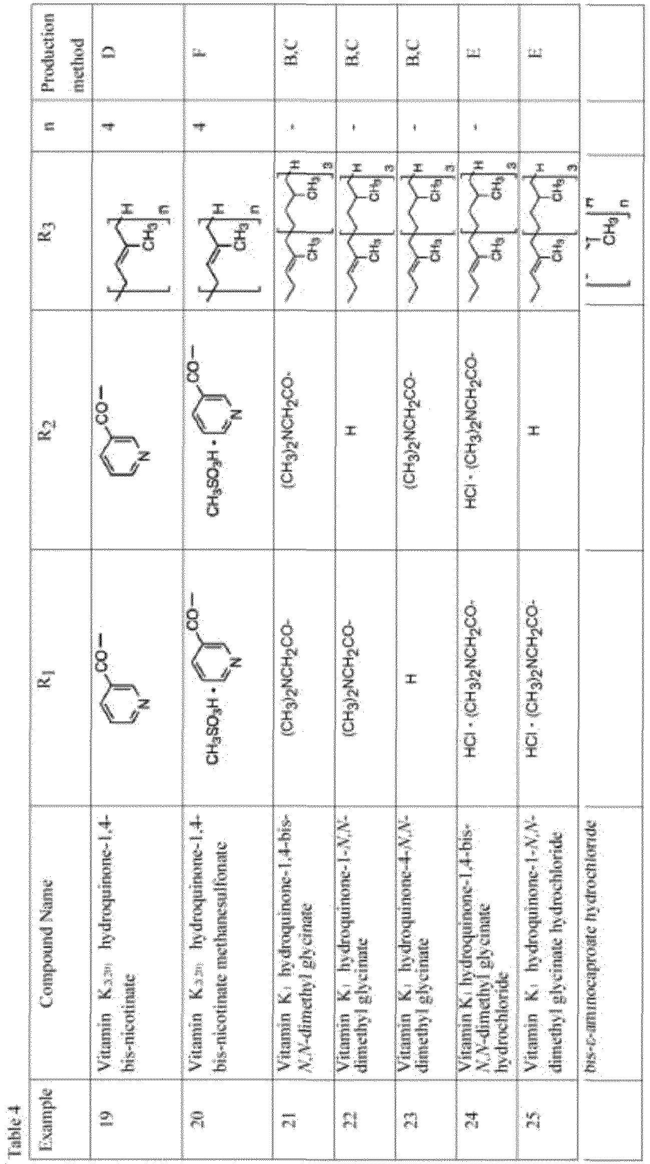

### [Table 5]

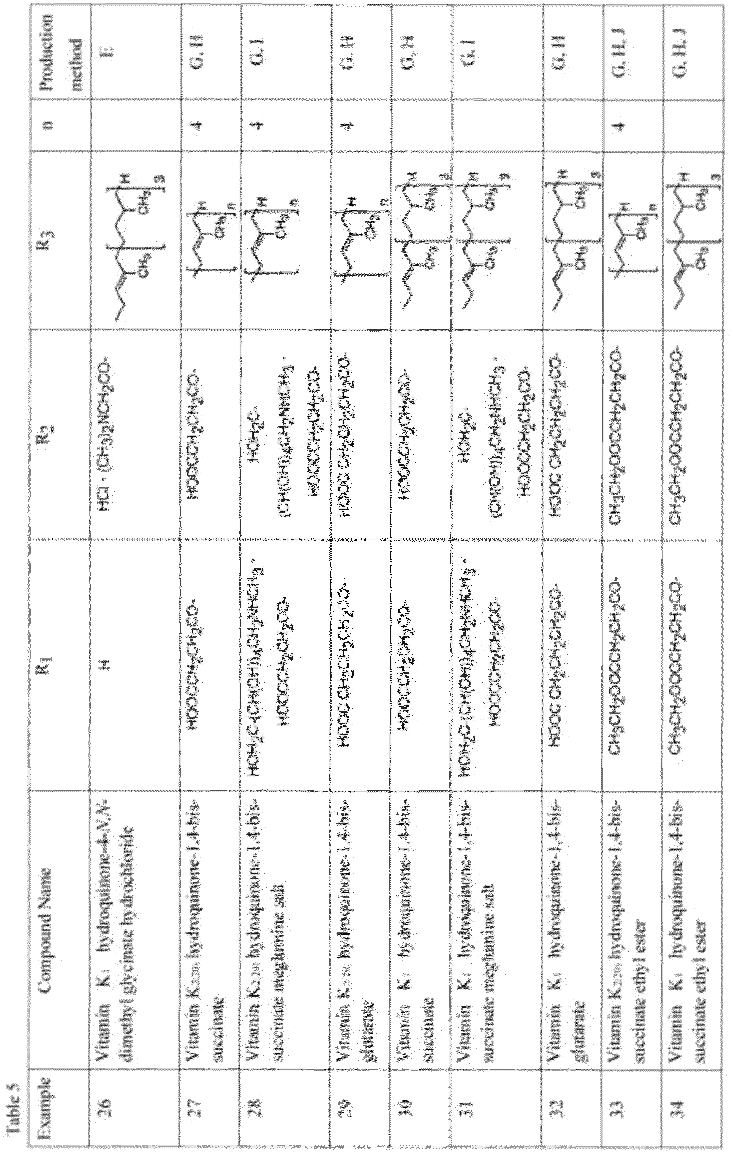

### [Table 6]

**Table 6**

| Example | Mass spectrometry | ¹H-NMR |
|---|---|---|
| | (m/z) | (δ ppm, internal standard TMS) |
| 1 | 760 (FD-MS) | ( in CDCl₃) |
| | (M⁺) | 7.70(2H,m),7.47(2H,m),5.15-5.01(6H,m),4.36(4H,s),3.40(2H,d),2.23(3H,s),2.05-1.93(12H,m), 1.77(3H.s),1.66-1.57(12H.m).1.48(18H,s) |
| 2 | 788 (FD-MS) | ( in CDCl₃) |
| | (M⁺) | 7.65(2H,m),7.46(2H,m),5.08(6H,m),3.57(4H,t),3.39(2H,d),3.01,(4H,m),2.23(3H,s), 2.05-1.95(12H,m),1.77(311,s),1.67-1.57(1.2H,m),1.47(18H,s) |
| 3 | 940 (FD-MS) | ( in CDCl₃) |
| | (M⁺) | 7.39-7.29(14H,m),5.09-4.99(8H,m),3.42(2H,d),3.26(4H,m),2.12(3H,s),2.06-1.93(12H,m), 1.73(3H,s),1.67-1.57(12H,m),1.44(9H,s),1.42(9H,s) |
| 4 | 788 (FD-MS) | |
| | (M⁺) | |
| 5 | 924 (FD-MS) | ( in CDCl₃) |
| | (M⁺) | 7.63(2H,m),7.43(2H,m),5.10-5.01(4H,m),4.62(2H,s),3.36(2H,s),3.04(4H,s),2.70(2H,m), 2.34(4H.m),2.19(3H,s),2.05-1.93(16H,m),1.75-1.46(39H,m).1.11(4H,m) |
| 6 | 872 (FD-MS) | (in CDCl₃) |
| | (M⁺) | 7.64(2H,m),7.44(2H,m),5.07(4H,m),4.51(2H,s),3.39(2H,d),3.16(4H,m),2.75(4H,m).2.21(3H.s), 2.07-1.85(16H.m),1.76(3H,s),1.67-1.45(38H,m) |
| 7 | 616 (FD-MS) | ( in CDCl₃) |
| | (M⁺) | 7.74(2H,m),7.50(2H,m),5.05(4H,m),3.72(4H,s),3.42(2H,d),2.47(6H,s),2.45(6H,s),2.23(3H,s), 2.09-1.90(12H,m),1.77(3H,s),1.64-1,52(12H,m). |
| 8 | 531 (FD-MS) | |
| | (M⁺) | |
| 9 | 531 (FD-MS) | |
| | (M⁺) | |
| 10 | 616 (FD-MS) | (in CDCl₃) |
| | (M⁺-2HCl) | 7.90(2H,m),7.61(2H,m),5.05(4H,m),4.90(4H,s),3.52(2H,d),3.13(6H,s),3.12(6H,s),2.32(3H,s), 2.10-1.89(12H,m),1.83(3H,s)1.63-1.51(12H,m) |

### [Table 7]

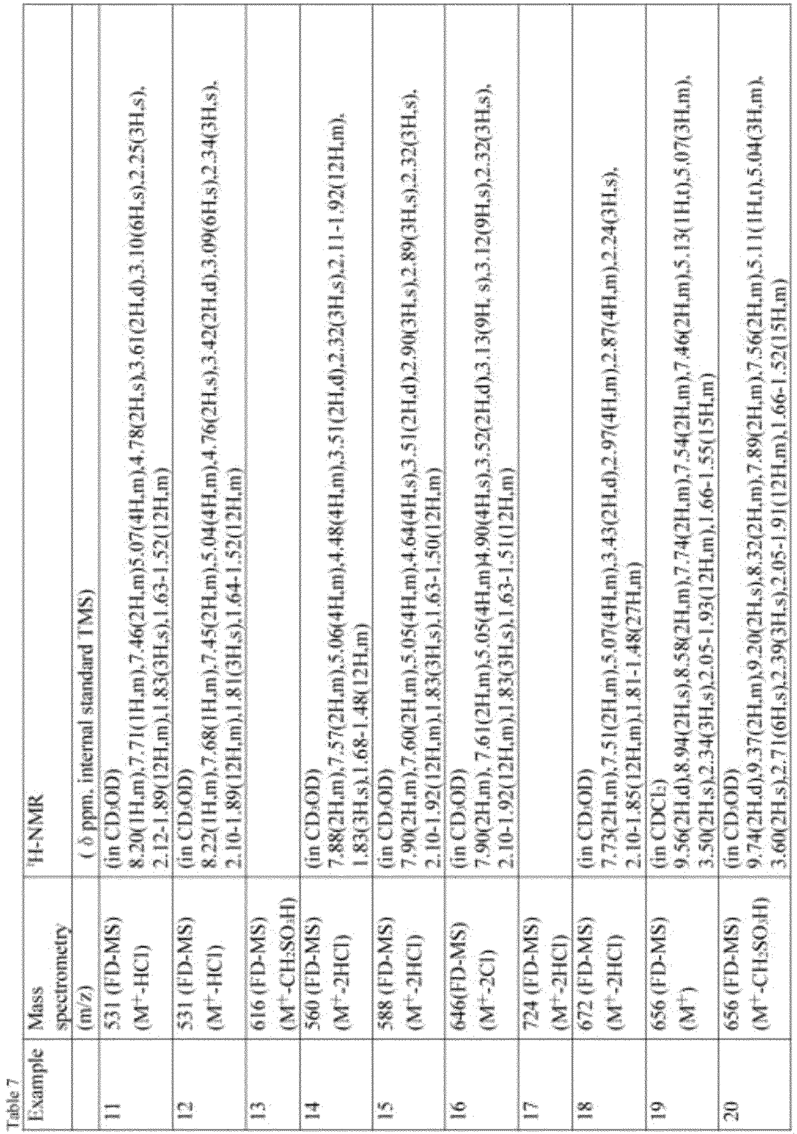

### [Table 8]

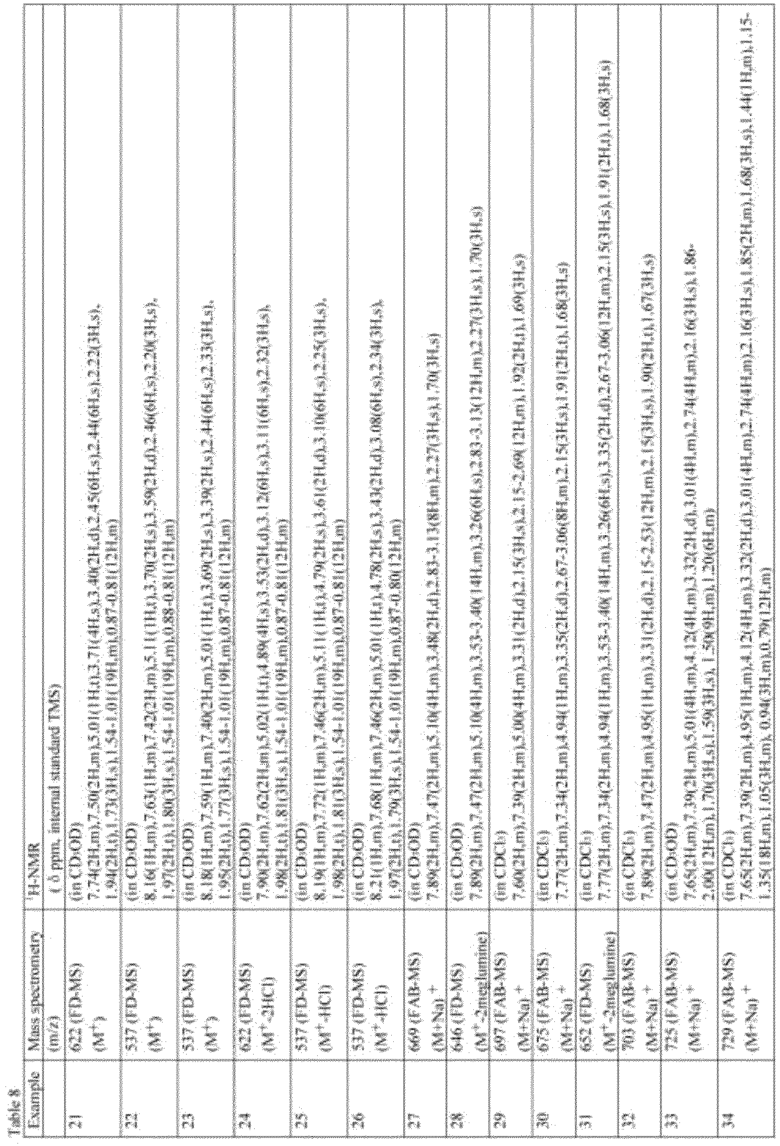

## Claims

1. A mitochondrial dysfunction improving agent comprising at least one of a carboxylic acid ester of an active vitamin K represented by a general formula (1) or a salt thereof. 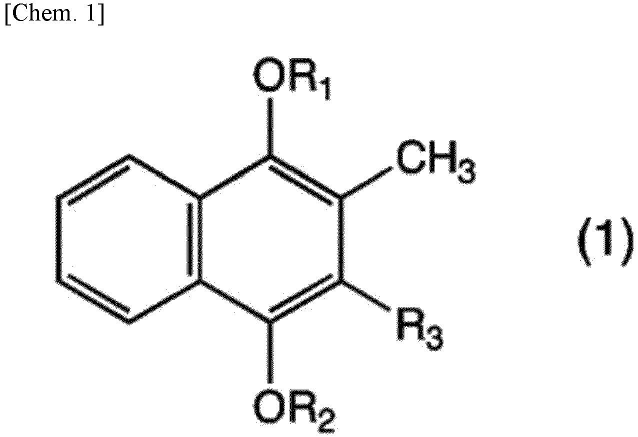 (wherein, R₁ and R₂ are a hydrogen atom, respectively, or a substituent selected from glycine, N-acyl glycine, N-alkyl glycine, N,N-dialkyl glycine, N,N,N-trialkyl glycine, acyl, dicarboxylic acid hemiester, and salts thereof; and at least either of R₁ and R₂ is glycine, N-acyl glycine, N-alkyl glycine, N,N-dialkyl glycine, N,N,N-trialkyl glycine, acyl, dicarboxylic acid hemiester, and salts thereof. R₃ is a group represented by a general formula (2), or a general formula (3). n is an integer of 1 to 7)

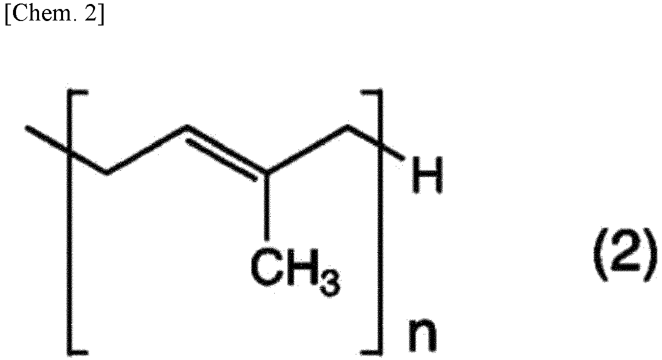

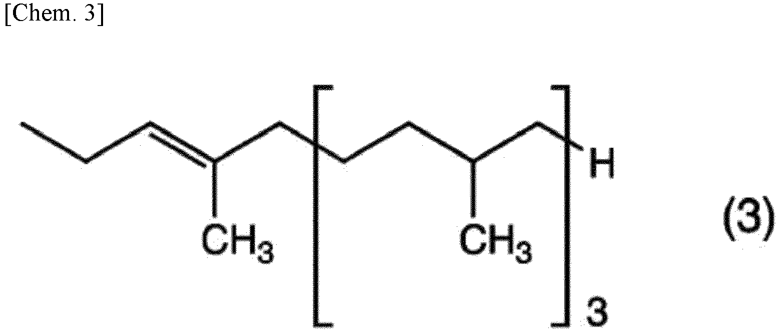

2. A carboxylic acid ester of an active vitamin K represented by a general formula (4) or a salt thereof. 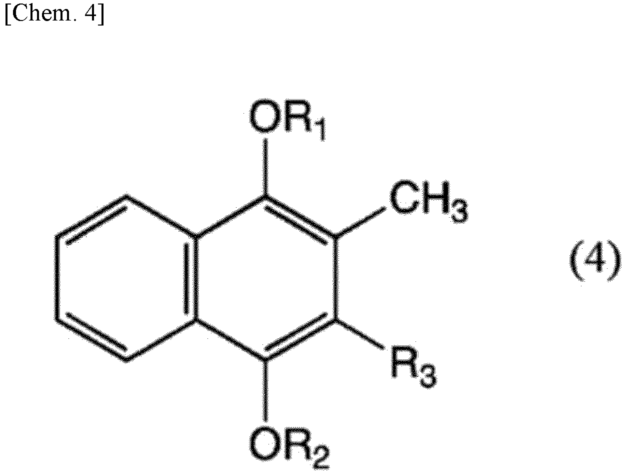 (in the general formula (4), R₁ and R₂ is a carboxylic acid residue selected from a group consisting of R₄OOCCH₂CH₂CO- and R₄OOCCH₂CH₂CH₂CO-. R₃ represents the above general formula (2) or (3). R₄ is a C1-C3 alkyl group.)

3. A neurodegeneration improving agent comprising the compound represented by the general formula (1) or a salt thereof.

4. An amyotrophic lateral sclerosis improving agent comprising the compound represented by the general formula (1) or a salt thereof.

5. An Alzheimer's disease improving agent comprising the compound represented by the general formula (1) or a salt thereof.

6. A Parkinson's disease improving agent comprising the compound represented by the general formula (1) or a salt thereof.

7. A neurodegeneration improving agent comprising the compound represented by the general formula (4) or a salt thereof.

8. An amyotrophic lateral sclerosis improving agent comprising the compound represented by the general formula (4) or a salt thereof.

9. An Alzheimer's disease improving agent comprising the compound represented by the general formula (4) or a salt thereof.

10. A Parkinson's disease improving agent comprising the compound represented by the general formula (4) or a salt thereof.
